# EUROPEAN PATENT SPECIFICATION

(11) **EP 1 568 356 B1**
(45) Date of publication and mention of the grant of the patent: **01.04.2009**
(21) Application number: 04075573.8
(22) Date of filing: 24.02.2004
(51) Int. Cl.: A61K 35/20, A61K 8/64, A61Q 11/00

(54) **Prevention of dental erosion by use of a colostrum protein**
Vorbeugung von Zahnerosion durch die Verwendung von Colostrumprotein
Prévention de l'érosion des dents par l'utilisation de protéine du colostrum

(43) Date of publication of application: 31.08.2005
(73) Proprietor: Sara Lee/DE N.V., 3532 AA Utrecht (NL)
(72) Inventor: Christoffersen, Jorgen, 2625 Vallensbaek (DK); Bardow, Allan, 2850 Gl. Holte (DK); Kirkeby, Svend, 1960 Frederiksenberg C (DK); Moe, Dennis, 2970 Horsholm (DK); Christoffersen, Margaret, 2625 Vallensbaek (DK); Nauntofte, Brigitte, 2920 Charlottenlund (DK); Pedersen, Anne marie, 2920 Charlottenlund (DK)
(74) Representative: Prins, Adrianus Willem

(56) References cited:
- EP-A- 0 743 060
- WO-A-02/061066
- US-A- 3 471 613
- DATABASE WPI Section Ch, Week 199014 Derwent Publications Ltd., London, GB; Class B04, AN 1990-103150 XP002288949 & JP 02 053716 A (LION CORP) 22 February 1990 (1990-02-22)
- LOIMARANTA V ET AL: "Generation of bovine immune colostrum against Streptococcus mutans and Streptococcus sobrinus and its effect on glucose uptake and extracellular polysaccharide formation by mutans streptococci." August 1997 (1997-08), VACCINE. AUG 1997, VOL. 15, NR. 11, PAGE(S) 1261 - 1268 , XP004086599 ISSN: 0264-410X * page 1268, left-hand column, last paragraph *

## Description

The invention relates to dental care and in particular to the use of a dental care product for preventing, or reducing dental erosion.

Dental care products are products that are intended for use in the oral cavity for some time having a cleaning, healing and/or prophylactic function, particular with a view to the health of the dentures (i.e. teeth and molars). They are generally not intended to be swallowed.

In the development of dental care products, much attention has been paid to various aspects of oral hygiene and oral care. The reduction of the number of bacteria in the oral cavity, the treatment of various mucous disorders and the prophylactic function of oral care products has been subject of much research and development.

Bacteria that are present in the oral cavity lead to dental plaque formation. Plaque is considered to be the prime etiological factor in the development of caries and it is also implicated in periodontal diseases. Plaque, when not sufficiently removed, develops into calculus, a hard mineralised formation which deposits on the enamel surface of the teeth. There is also an association between the presence of calculus and the incidence of periodontal diseases. Serious teeth problems, like caries, gingivitis and other periodontal diseases, can be the result of formation of plaque and calculus.

There are also other processes leading to tooth decay, which processes are not related to the presence of bacteria in the oral cavity. Dental erosion is defined as the loss of dental hard tissue by a chemical process not involving bacteria. Dental erosion constitutes a major problem in relation to dental health and its prevalence appears to be increasing. Soft drink consumption, and this extrinsic exposure to acid, has been shown to be one of the most important factors of dental erosion by Johansson et al., Acta Odontol. Scand., 1997, 55, 390-397. Generally, low pH in the oral cavity is believed to be the major cause of dental erosion.

The present invention seeks to mitigate the effects of dental erosion by providing a composition for a dental care product which reduces or prevents dental erosion. Surprisingly, it has been found that proteins present in colostrum, particularly bovine colostrum, may protect teeth from dental erosion. Accordingly, the invention relates to the use of a colostrum protein for the manufacture of a dental care product for preventing or reducing dental erosion.

Without wishing to be bound by any theory, it is contemplated that colostrum proteins may assist in the formation of a protective protein layer on the surface of the teeth. The formation of a layer of saliva proteins on the teeth is a well-known phenomenon. This layer is generally referred to as the pellicle. Not it is postulated that a protein obtainable from colostrum, in particular bovine colostrum, may be incorporated into the pellicle and thereby increase its protective strength, particularly to acid attack and thus to dental erosion.

In accordance with the invention, the term dental care product is used to indicate any form of product used by individuals or dental care professionals to treat the animal, but in particular, the human oral cavity for hygienic, prophylactic or therapeutic reasons. Specific examples include toothpastes or dentifrices, tooth creams, dental gels, mouth washes, chewing tablets or chewing gums, lozenges, effervescent tablets (which may require reconstitution in water), tooth powders and other suitable forms. It is preferred that the dental care product is a toothpaste or chewing gum.

Unless otherwise indicated, all weight percentages mentioned herein are based on the total weight of the dental care product.

Colostrum consists of a mixture of lacteal secretions and constituents of blood serum, notably immunoglobulins and other proteins, that accumulate in the mammary gland during the prepartum dry period. Colostrum can be harvested immediately preceding or following parturition. The first six milkings collected during the period of transition from colostrum to milk are typically still referred to as colostrum, in the case of cows as bovine colostrum. More particularly, colostrum is generally defined as the mammary secretion occurring in the period starting some days before delivery until some days after delivery.

Besides a protein fraction, colostrum comprises water, lactose and a fatty fraction. In accordance with the invention, it is important that at least the protein fraction of the colostrum is used in a dental care product. It is, however, not necessary to isolate the protein fraction; one or more of the other components of colostrum may also be present. Colostrum may be used in dried form, such as lyophilised or spray dried, or in the form of an extract, such as a concentrate or purified form. If desired, isolation of the protein fraction can be carried out in any known manner, which will typically include a degreasing step.

The colostrum used in accordance with the invention can be from any mammal, such as cows, sheep, goats and the like. It is preferred to use bovine colostrum, as this has been found to be particularly effective.

In the context of the invention, the term colostrum is intended to also include milk from animals, notably cows, which are treated with specific micro organisms such as S-mutans and other oral care selected pathogenic bacteria to produce a colostrum-type milk which is rich in immunoglobulins for longer periods (i.e. up to a year) after delivery. It is preferred, however, that normal, natural colostrum is used.

The amount in which a protein from colostrum is preferably used depends on the type of dental care product being prepared. Generally, the amount will lie within the range of from 0.1 to 50 wt.%, preferably from 0.5 to 25 wt.%. For toothpaste, the amount is preferably between 0.1 and 50 wt.%, more preferably between 0.1 and 15 wt.%; and for chewing gums the amount is preferably between 0.1 and 10 wt.%, more preferably between 0.1 and 5 wt.%.

A dental care product according to the invention may further comprise any conventional components usually present in dental care products, such as abrasives or polishing materials, thickening agents, colouring agents foaming agents, whitening agents, antibacterial proteins, neutralizing agents (bicarbonate), flavouring agents, sweeteners, preservatives and/or other basic ingredients. The choice for these components will depend on the actual nature of the dental care product.

In certain desirable forms of the invention, the dental care product may be substantially solid or pasty in character (tooth powders, dental tablets, toothpastes, or dental gels). Such solid or pasty preparations generally comprise an abrasive or polishing material.

Suitable examples of polishing materials include water-insoluble sodium metaphosphate, potassium metaphosphate, tricalcium phosphate, dehydrated calcium phosphate, anhydrous dicalcium phosphate, calcium pyrophosphate, magnesium orthophosphate, trimagnesium phosphate, aluminium oxide, aluminium oxide hydrate, calcium carbonate, aluminium silicate, zirconium silicate, silica, bentonite and mixtures thereof. Other suitable polishing materials include particulate thermosetting resins as disclosed in US patent 3,070,510, such as melamine-phenolic-, and urea-formaldehydes, and crosslinked polyepoxides and polyesters. Preferred polishing materials include silica having particle sizes of up to about 7 microns, a mean particle size of up to about 1.1 microns, and a surface area of up to about 50,000 cm²/gm, silica gel or colloidal silica, and complex amorphous alkali metal aluminosilicate.

When visually clear gels are desired, a polishing agent of colloidal silica, such as those sold under the trademark SYLOID as Syloid 72 and Syloid 74, or under the trademark SANTOCEL as Santocel 100 and alkali metal aluminosilicate complexes are particularly useful, since they have refractive indices close to the refractive indices of gelling agent-liquid (including water and/or humectant) systems commonly used in dental care products.

The polishing material is typically present in an amount ranging from 10 to 99 wt.%. Preferably, a polishing material is present in an amount from about 10 to about 75 wt.% in toothpaste, and from 70 to 99 wt.% in tooth powder.

In a toothpaste, the liquid vehicle may comprise water and humectant typically in an amount ranging from about 10 to about 90 wt.%. Glycerine, propylene glycol, sorbitol, polypropylene glycol, polyethylene glycol and mixtures thereof are typical examples of suitable humectants or carriers. Also advantageous are liquid mixtures of water, glycerine and sorbitol. In clear gels where the refractive index is an important consideration, about 3-30 wt.% of water, 0 to about 80 wt.% of glycerine, and about 20-80 wt.% of sorbitol is preferably employed.

Toothpastes and gels typically comprise a natural or synthetic thickener or gelling agent in proportions of about 0.1 to about 10 wt.%, preferably about 0.5 to about 5 wt.%. An example of a suitable thickener is synthetic hectorite, a synthetic colloidal magnesium alkali metal silicate complex clay available for examples under the trademark LAPONITE (e.g. CP, SP 2002, D) marketed by Laporte Industries Limited. Other suitable examples of thickeners include Irish moss, gum tragacanth, starch, polyvinyl pyrrolidone, hydroxyethyl propyl cellulose, hydroxybutyl methyl cellulose, hydroxypropyl methyl cellulose, hydroxyethyl cellulose (e.g. available as Natrosol), sodium carboxymethyl cellulose, and colloidal silica such as finely ground Syloid ™.

The vehicle or carrier in a tablet or lozenge is preferably a non-cariogenic solid water soluble polyhydric alcohol (polyol), such as mannitol, xylitol, sorbitol, maltitol, a hydrogenated starch hydrolysate, Lycasin, hydrogenated glucose, hydrogenated disaccharides, and hydrogenated polysaccharides, in an amount of about 90-98 wt.%. Solid salts such as sodium carbonate, sodium chloride, potassium bicarbonate, or potassium chloride may totally or partially replace the polyol carrier. Tabletting lubricants, in minor amounts of about 0.1 to 5 wt.%, may be incorporated into the tablet or lozenge formulation to facilitate the preparation of both the tablets and lozenges. Suitable lubricants include vegetable oils such as coconut oil, magnesium stearate, aluminium stearate, talc, starch and carbowax.

Formulation of a dental care product in the form of a lozenge or chewing gum may involve stirring any of the components set forth above into a warm gum base or coating the outer surface of a gum base. Illustrative of suitable gum bases are jelutone, rubber latex, vinylite resins, and the like, preferably in combination with conventional plasticizers or softeners, sugar or other sweeteners or carbohydrates such as glucose, sorbitol and the like.

Lozenge formulations may optionally comprise about 2 wt.% gum as barrier agent to provide a shiny surface as opposed to a tablet which has a smooth finish. Suitable non-cariogenic gums include Kappa carrageenan, carboxymethyl cellulose, hydroxyethyl cellulose, Gantrez™, and the like. The lozenge or tablet may optionally be coated with a coating material such as a wax, shellac, carboxymethyl cellulose, polyethylene maleic anhydride copolymer or Kappa carrageneean to further increase the dissolution time of the tablet or lozenge in the mouth. The uncoated tablet or lozenge is slow dissolving, providing a sustained release rate of the active ingredients. Accordingly, the solid dose tablet or lozenge compositions according to the invention afford a relatively longer contact time between active ingredients and oral tissues and structures.

Any suitable flavouring or sweetening material may also be employed. Examples of suitable flavouring constituents are flavouring oils, e.g. oil of spearmint, peppermint, wintergreen, sassafras, clove, sage, eucalyptus, marjoram, cinnamon, lemon, and orange, and methyl salicylate. Suitable sweetening agents include sucrose, lactose, maltose, sorbitol, xylitol, sodiuk cyclamate, perillartine, APM (aspartyl phenyl alanine methyl ester), saccharine, and the like. Suitably, flavouring and sweetening agents may together comprise from about 0.1 to about 5 wt.% or more of the dental care product.

A dental care product according to the invention may comprise one or more other materials that are considered as active components in oral care products, such as fluoride ion-providing agents, phosphates, such as trimetaphosphates and diammoniumphosphates, enzymes, anti-tartar agents such as pyrophosphates, phosphonates and/or other anti-plaque ingredients, such as triclosan, chlorhexidine, bromochlorophene and sanquinarine, antibacterial proteins, such as lanthibiotics (preferably nisin), agents for sensitive teeth, such as specific alkali metal salts like strontium salts and/or potassium nitrate or citrate, wound healing agents, such as allantoin, chlorophyll, tocopherol and herbal extracts, activity enhancing agents, or boosters, as Gantrez™ and some polymers, and specific ingredients as urea, xylitol, silicones, quaternary ammonium compounds and mixtures thereof. These components, as well as the amounts in which they may be used, are known in the art. If they are incorporated, they should be used in amounts which do not substantially adversely affect the properties and characteristics desired.

The presence of one or more fluoride ion-providing agents in a dental care product is particularly preferred. The use of fluoride ions, or fluoride ion providing compounds as anti-caries agents is well known in the art. Suitable compounds may be slightly soluble in water or may be fully water soluble. They are characterized by their ability to release fluoride ions in water and by freedom from undesired reaction with other compounds of a dental care product. Particularly, the use of alkali metal fluorides, more in particular sodium fluoride, results in improved activity of a dental care product.

Among suitable compounds yielding fluoride ions are inorganic fluoride slats, such as soluble alkali metal and alkaline earth metal salts, for example sodium fluoride, potassium fluoride, barium fluoride or calcium fluoride, and ammonium fluoride, or a copper fluoride, zinc fluoride, sodium fluorosilicate, ammonium fluorosilicate, sodium fluorozirconate, sodium monofluorphosphate, aluminium mono- or di-fluorophosphate and fluorinated sodium calcium pyrophosphate. Alkali metal and tin fluoride, such as sodium and stannous fluorides, sodium mono-fluorophosphate, organic fluorides, such as aminfluoride and mixtures thereof, are preferred.

The amount of fluoride ion-providing compounds is dependent to some extent upon the type of compound, its solubility, and the type of dental care product, but it must be a non-toxic, effective amount, generally about 0.005 to about 3.0 wt.% in the dental care product, an amount of such compound which releases up to about 5,000 ppm of fluoride ions by weight of the dental care product is considered satisfactory. Any suitable minimum amount of such compound may be used, but it is preferable to employ sufficient compound to release about 300 to 2,000 ppm, more preferably about 800 to about 1,500 ppm of fluoride ions. Typically, in the case of alkali metal fluorides and stannous fluoride, this component is present in an amount up to about 2 wt.%, and preferably in the range of about 0.05-1 wt.%. In the case of sodium mono-fluorophosphate, the component may be present in an amount of about 0.1-3 wt.%. In dental care products such as lozenges and chewing gums, the fluoride ion-providing compound is typically present in an amount sufficient to release up to about 500 ppm, preferably about 25 to about 300 ppm by weight of fluoride ions. Generally, about 0.005 to about 1.0 wt.% of such compound is present.

Enzymes that can be used to advantage in a dental care product according to the invention include lactoperoxidase and enzymes such as described in US patents 4,150,113, 4,178,362 and 4,781,532 and European patent application 0 133 736.

Lactoperoxidase is a glycoprotein which, in one commercial embodiment, is a lyophilised powder derived from milk, typically bovine milk. Lactoperoxidase is generally present in a dental care product according to the invention in an amount of about 0.2 to about 4.0 U per gram, and preferably of 2 U per gram of the dental care product.

Particular enzymes that are preferably used are in combination with or as alternative to lactoperoxidase are hydrogen peroxide producing enzyme systems, which have advantageous effects on the reduction of oral bacterial activity. Specific examples include oxidoreductases, such as glucose oxidase, galactose oxidase, urate oxidase, choline oxidase, D-amino acid oxidase, D-glutamate oxidase, glycine oxidase, glycollage oxidase, L-sorbose oxidase, alcohol oxidase, or amine oxidase, carbohydrases, such as α-amylase, glucoamylase or amylglucosidase, cellulase, dextranase, invertase, or α- or β-glucosidase, hydrolases and proteases. Preferably used are glucose oxidase, dextranase, lactoferrin, lysozyme, amyloglucosidase, and/or mutanase.

In order to enhance the activity of lactoperoxidase or of an oxdioreductase, if present, it is preferred that a dental care product according to the invention comprises a thiocyanate salt (SCN-). Preferred thiocyanate salts are alkali metal salts and alkaline earth metal salts, such as potassium thiocyanate or sodium thiocyanate. Thiocyanate salts are generally included in an amount in the range of from 0.005 to about 1 wt.%, dependent on the type of dental care product. For toothpastes, mouth washes and chewing gums the amount will preferably lie in the range of from 0.005 to 0.1 wt.%. For lozenges and effervescent tablets the amount preferably is in the range of from 1 to 10 mg/tablet.

The dental care product preferably further comprises zinc ions. These will generally be incorporated in the form of a salt. The anion with which the zinc ions form the salt may be any orally acceptable anion. Typical examples include fluoride, fluorosilicate, mono-fluorophosphate, chloride, citrate, gluconate, thiocyanate, sulphate, acetate and the like. Preferably, the anion is gluconate or citrate. The amount of zinc ions as Zn in a dental care product according to the invention is preferably in the range of from 0.0025 to 0.15 wt.%, more preferably from 0.01 to 0.1 wt.%, even more preferably from 0.025 to 0.05 wt.%. Of course, the weight percentage of the zinc salt incorporated into the dental care product will be larger, depending on the molecular weight of the anion in the salt.

The pH of a dental care product according to the invention is generally in the range of from about 4.5 to about 10, and preferably from about 5.5 to about 8. The pH can be controlled with acid (e.g. citric acid or benzoic acid) or base (e.g. sodium hydroxide), or be buffered using a neutralizing agent (e.g. sodium bicarbonate, citrate, benzoate, carbonate, disodium hydrogen phosphate, or sodium dihydrogen phosphate).

A dental care product can suitably be prepared using conventional methods and techniques for preparing such products, wherein the protein from colostrum is included during the preparation together with other active ingredients, or are added to the finished dental care product. Any relevant process parameters such as temperatures and pressures may suitably be chosen within conventional ranges.

The invention will now be further illustrated by the following, non-restrictive example.

### EXAMPLE

### Material and methods

Bovine colostrum protein was purified and degreased from a total of 5 liter colostrum (collected within 24 hours of the participating cow has calved). The bovine colostrum was collected in a glass container and out of contact with substances that could interfere with the experiments. As a control for the bovine colostrum human saliva was collected from 5 young healthy male volunteers and the saliva proteins purified. The saliva was collected directly from the parotid gland without exposure to the oral cavity. Thus the saliva proteins were not contaminated with substances originating from oral environment including the teeth.

The inhibiting effects of the bovine and saliva proteins on dissolution of human tooth substance (i.e. hydroxyapatite crystals denoted HAP) were determined. To establish a dose response relationship various concentrations of the proteins on HAP dissolution was determined.

The dissolution of HAP crystals was performed in an aqueous solution at pH 5.0. When HAP is exposed to water at this pH the pH will raise due to dissolution of the crystals. At this pH value each µM HAP will use 14 µM H⁺ to dissolve (i.e. Ca₁₀(PO₄)₆(OH)₂→ 10Ca²⁺ + 6H₂PO₄⁻ + 2H₂O). By continuous addition of acid (10 mM HNO₃) to maintain constant pH the dissolution was monitored as the amount of acid needed (µl/s). Differences in the amount of acid needed to maintain a constant pH with and without the proteins (colostrum and saliva) were regarded as the protective effects of the proteins on tooth dissolution. The protective effect (Jo/J) was calculated as the amount of acid needed without the proteins (Jo) divided by the amount of acid needed with the proteins (J) as previously described (Christoffersen et al., Colloids and Surfaces 1986, 18, 1-8).

### Results

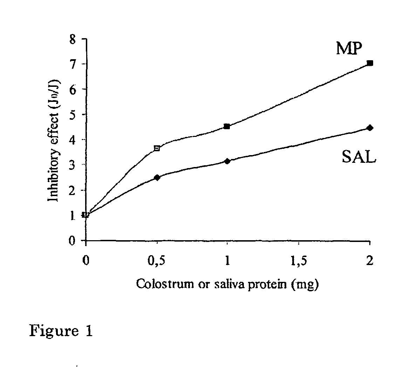

Figure 1 shows the inhibitory effect (Jo/J) of colostrum and saliva proteins on the dissolution of 14 mg HAP crystals in 500 ml aqueous solution approximately 79% saturated with respect to HAP at pH 5 and 37°C. Here (MP) denotes colostrum (i.e. milk proteins) and (SAL) the mean protective of the saliva proteins from the five individuals. As shown in figure 1 a dose response relationship was established between the concentration of the proteins and the inhibitory effect becoming higher with higher concentrations of the proteins. Furthermore, colostrum proteins were twice as effective as saliva proteins in inhibiting HAP dissolutions in all three concentrations (i.e. 0.5, 1.0, and 2.0 mg).

### Perspectives and implementation

Our results showed that colostrum proteins in the concentrations 0.5, 1.0, and 2.0 inhibited the dissolution of HAP crystals at pH by a factor of up to seven times. The results also showed that the colostrum proteins were better inhibitors of HAP dissolution than the saliva proteins that normally cover the teeth in the mouth.

The surface area of the 14 mg of HAP crystals used was 4900 cm², which gives a ratio between the HAP surface area and colostrum protein of 0.41 µg protein per cm² (in the experiment with the 2 mg protein concentration). This ratio is far lower than what can be expected in the mouth after brushing teeth with toothpaste containing 1% colostrum proteins. Thus the average surface area of a full human dentition is 70 cm² (see also Chase, J. Am. Dent. Assoc., 1927, 14, 491-492) and the average weight of one portion toothpaste is 1 g (10 mg of colostrum protein) giving rise to a surface area colostrum protein ratio of 143 µg per cm². Accordingly the expected amount of colostrum protein available per cm² of tooth surface during and after tooth brushing is expected to be nearly 350 times higher than the ratios used in this in vitro experiment.

Thus the inhibitory effect of colostrum proteins added to tooth paste in a concentration of 1% will inhibit HAP (i.e. tooth) dissolution and thus dental erosion also in vivo in the mouth. The inhibitory effect in the mouth is expected to be higher than the seven times inhibition obtained in this *in vitro* study.

## Claims

1. Use of a colostrum protein for the manufacture of a dental care product for preventing or reducing dental erosion.

2. Use according to claim 1, wherein the dental care product comprises a fluoride providing agent.

3. Use according to claim 1 or 2, wherein the dental care product further comprises one or more phosphates, enzymes, anti-tartar agents, agents for sensitive teeth, wound healing agents, activity enhancing agents or boosters, urea, xylitol, silicones, quaternary ammonium compounds and combinations thereof.

4. Use according to any of the preceding claims, wherein the dental care product comprises from 0.1 to 50 wt.%, preferably from 0.5 tot 25 wt.%, based on dry weight of the dental care product, of the colostrum protein.

5. Use according to any of the preceding claims, wherein the dental care product further comprises an abrasive agent, a polishing agent, a thickening agent, a colouring agent, a sweetening agent, a flavouring agent, a foaming agent, a bactericidal agent, or a combination thereof.

6. Use according to any of the preceding claims, wherein the dental care product has the form of a tooth paste or cream, dental gel, tooth powder, mouthwash, chewing gum, dental or chewing tablet, lozenge or effervescent tablet.

7. Use according to claim 6, wherein the dental care product is in the form of a tooth paste or gel and further comprises a humectant.

8. Use according to any of the preceding claims, wherein the dental care product further comprises zinc ions.

## Patentansprüche

1. Verwendung eines Colostrumproteins zur Herstellung eines Zahnpflegeprodukts zur Prävention oder Reduktion der Zahnerosion.

2. Verwendung gemäß Anspruch 1, wobei das Zahnpflegeprodukt ein fluoridlieferndes Mittel umfasst.

3. Verwendung gemäß Anspruch 1 oder 2, wobei das Zahnpflegeprodukt weiterhin ein oder mehrere Phosphate, Enzyme, Antizahnsteinmittel, Mittel für empfindliche Zähne, Wundheilungsmittel, aktivitätssteigernde Mittel oder Booster, Harnstoff, Xylit, Silikone, quartäre Ammon'iumverbindungen und Kombinationen davon umfasst.

4. Verwendung gemäß einem der vorstehenden Ansprüche, wobei das Zahnpflegeprodukt 0,1 bis 50 Gew.-%, vorzugsweise 0,5 bis 25 Gew.-%, des Colostrumproteins umfasst, bezogen auf das Trockengewicht des Zahnpflegeprodukts.

5. Verwendung gemäß einem der vorstehenden Ansprüche, wobei das Zahnpflegeprodukt weiterhin ein abrasives Mittel, ein polierendes Mittel, ein Verdickungsmittel, eine Färbemittel, ein Süßungsmittel, einen Aromastoff, ein Schäumungsmittel, ein bakterizides Mittel oder eine Kombination davon umfasst.

6. Verwendung gemäß einem der vorstehenden Ansprüche, wobei das Zahnpflegeprodukt in Form einer Zahnpasta oder -creme, eines Zahngels, Zahnpulvers, einer Mundspülung, eines Kaugummis, einer Zahn- oder Kautablette, Pastille oder Brausetablette vorliegt.

7. Verwendung gemäß Anspruch 6, wobei das Zahnpflegeprodukt in Form einer Zahnpasta oder eines Zahngels vorliegt und weiterhin ein Feuchthaltemittel umfasst.

8. Verwendung gemäß einem der vorstehenden Ansprüche, wobei das Zahnpflegeprodukt weiterhin Zinkionen umfasst.

## Revendications

1. Utilisation d'une protéine de colostrum pour la fabrication d'un produit de soin dentaire pour prévenir ou réduire l'érosion dentaire.

2. Utilisation selon la revendication 1, dans laquelle le produit de soin dentaire comprend un agent fournissant du fluor.

3. Utilisation selon la revendication 1 ou 2, dans laquelle le produit de soin dentaire comprend en outre un ou plusieurs phosphates, enzymes, agents anti-tartre, des agents pour dents sensibles, agents de guérison des plaies, agents amplifiant l'activité ou stimulants, de l'urée, du xylitol, silicones, composés ammonium quaternaire et combinaisons de ceux-ci.

4. Utilisation selon l'une des revendications précédentes, dans laquelle le produit de soin dentaire comprend de 0,1 à 50 % en poids, de préférence de 0,5 à 25 % en poids de protéine de colostrum, sur la base du poids sec du produit de soin dentaire.

5. Utilisation selon l'une quelconque des revendications précédentes, dans laquelle le produit de soin dentaire comprend en outre un agent abrasif, un agent de polissage, un agent épaississant, un agent colorant, un agent édulcorant, un agent aromatisant, un agent moussant, un agent bactéricide, ou une combinaison de ceux-ci.

6. Utilisation selon l'une des revendications précédentes, dans laquelle le produit de soin dentaire a la forme d'une pâte de dentifrice ou d'une crème, d'un gel dentaire, d'une poudre dentaire, d'un rince-bouche, d'un chewing gum, d'une tablette à mâcher ou dentaire, d'une pastille ou d'une tablette effervescente.

7. Utilisation selon la revendication 6, dans laquelle le produit de soin dentaire est sous la forme d'une pâte de dentifrice ou d'un gel et comprend en outre un humidifiant.

8. Utilisation selon l'une des revendications précédentes, dans lequel le produit de soin dentaire comprend en outre des ions zinc.
